# FASCICULE DE BREVET EUROPEEN

(11) **EP 0 988 086 B1**
(45) Date de publication et mention de la délivrance du brevet: **07.07.2004**
(21) Numéro de dépôt: 98920584.4
(22) Date de dépôt: 08.04.1998
(51) Int. Cl.: A61N 1/32

(54) **SYSTEME D'APPLICATION D'OR AUX TISSUS CUTANES DU VISAGE**
Anordnung zum Auftragen von Gold auf die Gesichtshaut
SYSTEM FOR APPLYING GOLD TO THE FACE SKIN TISSUES

(43) Date de publication de la demande: 29.03.2000
(73) Titulaire: Parienti, Raoul, 06000 Nice (FR)
(72) Inventeur: Parienti, Raoul, 06000 Nice (FR)
(74) Mandataire: Bonneau, Gérard
(86) Numéro de dépôt international: PCT/FR1998/000706
(87) Numéro de publication internationale: WO 1998/046302

(56) Documents cités:
- EP-A- 0 547 482
- EP-A- 0 778 046
- DE-A- 2 828 936
- FR-A- 2 336 949
- FR-A- 2 661 616
- US-A- 4 317 457
- US-A- 4 405 311
- US-A- 4 411 648
- US-A- 5 443 441

## Description

### Domaine technique

La présente invention concerne un système permettant l'application d'atomes d'or sur le visage dans le but de remodeler les tissus pour faire disparaître les ridules du visage.

### Etat de la technique

Depuis la plus haute antiquité l'or est connu pour ses vertus thérapeutiques bénéfiques. En outre, depuis plus d'une décennie, la pharmacopée fait de plus en plus usage des vertus régénératrices tonifiantes et bactéricides de l'or administré par voie orale ou perlinguale.

Depuis une vingtaine d'années, le développement en occident des techniques traditionnelles chinoises telles que l'acupuncture a mis en évidence que de très fines aiguilles d'or insérées dans les ridules provoquaient une réduction sensible de ces dernières et un remodelage des tissus adjacents donnant au visage une bonne mine durable. Toutefois cette façon de pratiquer ne permet pas une application homogène et ne convient pas à certaines personnes qui ne supportent pas l'introduction d'aiguilles dans la peau.

### Exposé de l'invention

Le but de l'invention est donc principalement de fournir un système permettant l'application d'atomes d'or sur la peau du visage de façon homogène ne nécessitant pas l'introduction d'aiguilles.

L'invention concerne donc un système d'application d'or aux tissus cutanés du visage d'un patient comprenant un dispositif de génération de tension unipolaire, un masque dont la paroi intérieure est recouverte d'or et destiné à être placé sur le visage du patient par l'intermédiaire d'un fluide du type gel jouant le rôle d'électrolyte en contact d'une part avec la peau du visage et d'autre part avec la paroi intérieure du masque connecté à la borne positive du dispositif de génération de tension, et une électrode en contact avec une partie cutanée du corps du patient et connectée à la borne négative du dispositif de génération de tension de manière à ce que des atomes d'or soient transférés du masque à la peau du patient par électrolyse à travers le fluide servant d'électrolyte.

### Description de l'invention

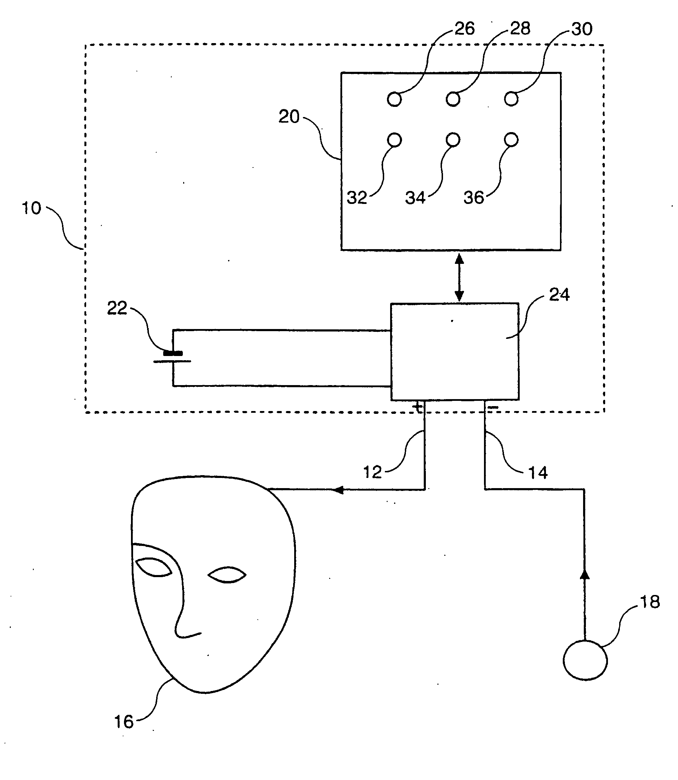

Les buts, objets et caractéristiques de l'invention apparaîtront mieux à la lecture de la description suivante faite en référence à la figure unique jointe.

Un système selon l'invention comprend essentiellement un boîtier de commande 10 en tant que dispositif de génération de tension unipolaire représenté en pointillés sur la figure et comportant deux bornes de sortie, une borne positive 12 connectée à un masque 16 servant d'anode et une électrode négative 18 en tant que cathode.

Le boîtier de commande 10 comprend une unité de commande 20 telle qu'un microprocesseur, une source d'alimentation 22 fournissant une faible tension continue de préférence comprise entre 3 et 6 volts et un convertisseur/applicateur de tension 24 permettant d'obtenir une tension voulue aux bornes de sortie 12 et 14.

Le masque 16 est de préférence en une matière souple telle qu'un polymère, enrichie de poudre de carbone ou autre matière conductrice pour le rendre conducteur. Sa paroi intérieure est recouverte d'une mince pellicule d'or, par exemple de 1 ou 2 microns d'épaisseur, obtenue par électrolyse ou tout autre procédé de dorure. L'essentiel est qu'il y ait un contact intime entre le support souple du masque et la pellicule d'or pour que la connexion avec la borne positive 12 soit bien réalisée.

Avant d'appliquer le masque sur le visage d'un patient, le visage est enduit d'un gel ou tout autre fluide utilisé comme électrolyte. Ce dernier contient un mélange d'acides (par exemple acide nitrique et acide sulfurique) contenant des ions d'or en solution. Les acides doivent être dilués pour ne pas attaquer la peau, une dilution entraînant un pH égal à 4 ou 5 étant adéquate pour obtenir un bon électrolyte. L'électrolyte pourrait être tout autre solution contenant des ions d'or comme par exemple de l'eau de mer qui, bien qu'elle contienne déjà de l'or sous forme d'ions (4g/m³), demande à être enrichie en atomes d'or.

L'emploi d'un gel de préférence à tout autre fluide, présente l'avantage de bien s'adapter à la forme du visage lorsque le masque 16 est appliqué. Un tel gel peut être un gel silicone ou un gel acrylique.

La borne négative 14 est connectée à la cathode 18 qui est de préférence sous forme d'un bracelet conducteur attaché autour d'un des poignets du patient.

Comme illustré sur la figure, le microprocesseur 20 connecté au convertisseur/applicateur de tension 24 comprend plusieurs boutons de commande 26 à 36 destinés à faire varier les différents paramètres de commande du système. Ainsi, le bouton 26 commande le temps d'application (par exemple de 15 mn à 30 mn), le bouton 28 commande la valeur de tension appliquée entre les bornes 12 et 14, et le bouton 30 commande la forme ou la modulation de la tension appliquée. Les autres boutons de commande pourraient être en relation avec le patient. Ainsi, le bouton 32 peut indiquer l'âge dans la mesure où la tension à appliquer est fonction de l'âge, le bouton 34 peut indiquer le sexe dans la mesure où la tension à appliquer diffère selon qu'il s'agit d'un homme ou d'une femme et le bouton 36 peut indiquer l'état de la peau (sèche, humide, grasse...).

De façon générale, le microprocesseur peut disposer d'autant de commandes qu'il est nécessaire, mais également d'un écran à cristaux liquides pour afficher par exemple la tension appliquée.

De façon à améliorer l'application d'or sur la peau du patient, la tension entre les deux bornes de sortie 12 et 14 peut avoir une valeur variable, mais toujours de même polarité. Ainsi, cette tension peut se présenter sous forme d'impulsions positives telles que des créneaux ou une suite d'alternances sinusoïdales d'une tension alternative redressée.

L'application de tension pendant une période d'environ 20 mn permet l'apport de quelques microgrammes d'or qui se déposent sur la partie sous-cutanée du visage. De façon à déboucher les pores de la peau qui pourraient se trouver obstrués après le passage du courant, il est bon d'inverser celui-ci pendant quelques instants à la fin de la période d'application. A la fin de l'application d'or, et si l'épaisseur de la couche d'or du masque a été réglée correctement, il ne doit plus y avoir d'or sur le masque qui retrouve alors son aspect d'origine avant d'avoir été doré.

## Revendications

1. Système d'application d'or aux tissus cutanés du visage comprenant un dispositif de génération de tension unipolaire (10) dont la borne positive (12) est connectée à une première électrode telle qu'un masque (16) destinée à être placée sur le visage d'un patient par l'intermédiaire d'un fluide et dont la borne négative (14) est connectée à une seconde électrode (18) adaptée à être mise en contact avec une partie cutanée du corps du patient ; ledit système étant **caractérisé en ce que** :
la paroi intérieure de ladite première électrode est recouverte d'or et ledit fluide est du type gel jouant le rôle d'électrolyte en contact avec la paroi intérieure de ladite première électrode de manière à ce que des atomes d'or soient transférés de ladite première électrode à la peau du patient par électrolyse à travers ledit fluide.

2. Système selon la revendication 1, dans lequel ledit dispositif de génération de tension unipolaire (10) comprend :
une source d'alimentation (26) fournissant une tension continue,
un convertisseur/applicateur de tension (24) disposant d'une borne de sortie positive (12) connectée à ladite première électrode telle qu'masque (16) et d'une borne de sortie négative (14) connectée à ladite seconde électrode (18) pour fournir ladite tension unipolaire, et
un microprocesseur (20) connecté audit convertisseur/applicateur de tension pour contrôler les paramètres de détermination de ladite tension unipolaire.

3. Système selon la revendication 2, dans lequel ledit microprocesseur (20) comporte plusieurs boutons de commande dont un bouton de commande du temps d'application (26), un bouton de commande de la valeur de la tension appliquée (28) et un bouton de commande de la forme de la tension appliquée (30).

4. Système selon la revendication 3, dans lequel ledit microprocesseur (20) comprend en outre un bouton définissant l'âge du patient (32), un bouton définissant le sexe du patient (34) et un bouton définissant l'état de la peau du visage du patient (36).

5. Système selon l'une des revendications 2 à 4, dans lequel ladite tension unipolaire fournie par ledit convertisseur/applicateur (24) a une valeur comprise entre 3 et 6 volts.

6. Système selon l'une des revendications 2 à 5, dans lequel ladite tension unipolaire fournie par ledit convertisseur/applicateur (24) est sous forme d'impulsions positives.

7. Système selon l'une des revendications 2 à 5, dans lequel ladite tension unipolaire fournie par ledit convertisseur/applicateur (24) est inversée pendant une courte période à la fin du temps d'application normal.

## Patentansprüche

1. System zum Auftragen von Gold auf die Gewebe der Gesichtshaut, bestehend aus einer Vorrichtung zur Erzeugung unipolarer Spannung (10), deren positive Polklemme(12) angeschlossen wird an eine erste Elektrode wie eine Maske (16), welche über ein Medium auf das Gesicht eines Patienten aufgelegt wird, und deren negative Polklemme (14) angeschlossen wird an eine zweite Elektrode (18), welche dazu dient, in Berührung mit einer Hautpartie des Patientenkörpers gebracht zu werden; welches System **dadurch gekennzeichnet ist, dass**:
die Innenwand der besagten ersten Elektrode goldbedeckt ist und das besagte Medium ein Gel ist, welches die Funktion eines Elektrolyten in Berührung mit der besagten Innenwand übernimmt, so dass Goldatome mittels Elektrolyse durch das besagte Medium von der besagten ersten Elektrode auf die Haut des Patienten übertragen werden.

2. System nach Anspruch 1, bei welchem die besagte Vorrichtung zur Erzeugung unipolarer Spannung (10) folgende Bauteile umfasst:
eine Versorgungsquelle (26) für Gleichspannung,
einen Spannungswandler/-applikator (24) mit einer positiven Ausgangsklemme (12), welche angeschlossen wird an die besagte erste Elektrode wie eine Maske (16), und mit einer negativen Ausgangsklemme (14), welche angeschlossen wird an die besagte zweite Elektrode (18), um die besagte Gleichspannung zu liefern; und
einen Mikroprozessor (20), welcher angeschlossen wird an den besagten Spannungswandler/-applikator, um die Bestimmungsparameter der besagten unipolaren Spannung zu kontrollieren.

3. System nach Anspruch 2, bei welchem der besagte Mikroprozessor (20) mehrere Bedienknöpfe aufweist, darunter einen Bedienknopf für die Auftragungszeit (26), einen Bedienknopf für den Wert der angelegten Spannung (28) und einen Bedienknopf für die Form der angelegten Spannung (30).

4. System nach Anspruch 3, bei welchem der besagte Mikroprozessor (20) außerdem einen Knopf für das Alter des Patienten (32), einen Knopf für das Geschlecht des Patienten (34) und einen Knopf für den Zustand der Gesichtshaut des Patienten (36) aufweist.

5. System nach einem der Ansprüche 2 bis 4, bei welchem die besagte vom Wandler/Applikator (24) gelieferte unipolare Spannung einen Wert zwischen 3 und 6 Volt besitzt.

6. System nach einem der Ansprüche 2 bis 5, bei welchem die besagte vom Wandler/Applikator (24) gelieferte unipolare Spannung die Form positiver Impluse besitzt.

7. System nach einem der Ansprüche 2 bis 5, bei welchem die besagte vom Wandler/Applikator (24) gelieferte unipolare Spannung am Ende der normalen Auftragungszeit kurzzeitig umgekehrt wird.

## Claims

1. A system for applying gold to facial skin tissues comprising a single-pole voltage generating device (10) the positive terminal (12) of which is connected to a first electrode such as a mask (16) aimed at being placed on a patient's face by the intermediary of a fluid and the negative terminal (14) of which is connected to a second electrode (18) aimed at being in contact with a cutaneous part of the patient's body, said mask being connected to the positive terminal (12) of said voltage generating device, and
**characterized in that** the internal surface of said first electrode is covered with gold and said fluid is a gel-type fluid which acts as an electrolyte in contact with the internal surface of said first electrode so that the gold atoms are transferred from said first electrode to the patient's skin by electrolysis through said fluid.

2. The system according to claim 1, wherein said single-pole voltage generating device (10) comprises:
a power supply source (26) delivering a continuous voltage,
a voltage converter/supply (24) provided with a positive output terminal (12) connected to said mask (16) and a negative output terminal (14) connected to said patient's electrode, for supplying said single-pole voltage, and
a microprocessor (20) connected to said voltage converter/supply for controlling the settings that determine said single-pole voltage.

3. The system according to claim 2, wherein said microprocessor (20) has several control buttons including a control button (26) for setting the duration of the application, a control button (28) for setting the voltage value applied and a control button (30) for setting the shape of the voltage applied.

4. The system according to claim 3, wherein said microprocessor (20) further includes a control button (32) for setting the patient's age, a control button (34) for setting the patient's sex, and a control button (36) for setting the condition of the patient's facial skin.

5. The system according to any one of claims 2 to 4, wherein the value of said single-pole voltage provided by said converter/supply (24) ranges from 3 to 6 Volts.

6. The system according to any one of claims 2 to 5, wherein the value of said single-pole voltage provided by said converter/supply (24) has the form of positive pulses.

7. The system according to any one of claims 2 to 5, wherein the value of said single-pole voltage provided by said converter/supply (24) is reversed for a short period at the end of the normal application duration.
